# EUROPÄISCHE PATENTSCHRIFT

(11) **EP 4 202 182 B1**
(45) Veröffentlichungstag und Bekanntmachung des Hinweises auf die Patenterteilung: **13.08.2025**
(21) Anmeldenummer: 22211914.1
(22) Anmeldetag: 07.12.2022
(51) Int. Cl.: F01C 21/10, F04C 2/18, F04C 2/08

(54) **VERFAHREN ZUM HERSTELLEN EINER PUMPE**
METHOD FOR PRODUCING A PUMP
PROCÉDÉ DE FABRICATION D'UNE POMPE

(30) Priorität: 16.12.2021 DE 102021133396
(43) Veröffentlichungstag der Anmeldung: 28.06.2023
(73) Patentinhaber: B. Braun Avitum AG, 34212 Melsungen (DE)
(72) Erfinder: WÜRSCHMIDT, Tobias, 34346 Hann. Münden (DE)
(74) Vertreter: Winter, Brandl - Partnerschaft mbB

(56) Entgegenhaltungen:
- WO-A2-2012/066026
- DE-A1- 102019 102 073
- US-A- 3 986 797
- US-A1- 2002 021 979

## Beschreibung

### Technisches Gebiet

Die vorliegende Offenbarung betrifft ein Verfahren zur Herstellung einer Pumpe, eine Pumpe zur Verwendung in einer Dialysemaschine und eine Dialysemaschine zur extrakorporalen Behandlung von Blut.

### Hintergrund der Offenbarung

Zahnradpumpen sind weitverbreitete Flusspumpen und seit langer Zeit im Einsatz. Bei einer bekannten Zahnradpumpe wird ein Zahnrad auf einer Welle angetrieben. Das antreibende Zahnrad treibt ein zweites Zahnrad an. Beide Zahnräder laufen dabei in zylindrischen Zahnradaufnahmen. Zwischen einer Wand der Zahnradaufnahme und den Zahnrädern ist jeweils ein kleiner Spalt vorhanden. Durch die Bewegung der Zahnräder wird eine Flüssigkeit befördert. Entscheidend für eine Förderrate und einen Förderdruck der Zahnradpumpe sind ein Zahnraddurchmesser und/oder eine Zahnradbreite. D.h. die Förderrate und/oder der Förderdruck werden über eine Variation des Zahnraddurchmessers, der Zahnradbreite oder beides eingestellt.

Es ist bekannt, Zahnradpumpen in medizinischen Geräten, wie Dialysemaschinen einzusetzen. In bekannten Dialysemaschinen werden drei verschiedene Arten von Zahnradpumpen in einem Dialysierflüssigkeitskreislauf eingesetzt. Mit einer Entgasungspumpe wird die in die Dialysemaschine eingeleitete Dialysierflüssigkeit entgast. Eine Dialysierflüssigkeit-Eingangspumpe und eine Dialysierflüssigkeit-Ausgangspumpe werden zur Bilanzierung der Dialysebehandlung verwendet. Dabei sind die verschiedenen Zahnradpumpen für unterschiedliche Flussbereiche ausgelegt. So muss die Entgasungspumpe ein höheres Fördervolumen bzw. einen höheren Volumenstrom liefern als die Dialysierflüssigkeit-Eingangspumpe und die Dialysierflüssigkeit-Ausgangspumpe. D.h. die Entgasungspumpe muss eine höhere Leistung als die Dialysierflüssigkeitspumpen bereitstellen. Daher weist die Entgasungspumpe in bekannten Dialysemaschinen dickere Zahnräder als die Dialysierflüssigkeit-Ein- und Ausgangspumpen auf. Die Zahnraddurchmesser sind bei allen drei Zahnradpumpen identisch. An der Dialysierflüssigkeit-Eingangspumpe ist ein Rückschlagventil verbaut, das bei einem Überdruck öffnet und die Dialysemaschine vor zu hohen Drücken schützt.

Durch die höhere Zahnraddicke der Entgasungspumpe muss eine Aufnahmetasche für die Zahnräder dieser Pumpe tiefer sein als bei den anderen Zahnradpumpen. Da sich alle drei Zahnradpumpen unterscheiden, weist jede der Zahnradpumpen einen eigenen Pumpengehäuseabschnitt auf, in dem die Zahnräder gelagert sind. Herkömmlich werden die drei unterschiedlichen Pumpengehäuseabschnitte aus einem rohen Rundstahl gefräst bzw. spanend bearbeitet. Ein eigener unterschiedlicher Pumpengehäuseabschnitt für jede der Variationen der Zahnradpumpen spanend herzustellen, hat die folgenden Nachteile:
- Für jede Pumpe werden neue teure Rundmaterialien benötigt
- Aufwendige Bearbeitung
- Tüllenmontage erforderlich
- Viel Abfall
- Keine optimale Ressourcen Ausnutzung.

Die gefrästen Pumpengehäuseabschnitte haben keine Schlauchtüllen, die zum Anschluss von Schläuchen dienen. Diese müssen in einem eigenen Montageschritt montiert werden. Das bringt einen hohen manuellen Montageaufwand mit sich. Es ist ferner möglich, nicht einzelne Pumpengehäuseabschnitte zu fertigen, sondern die unterschiedlichen Zahnraddicken mit unterschiedlich dicken Distanzringen bzw. Dichtplatten einzustellen. D.h. das Pumpengehäuse bleibt das gleiche und die unterschiedlichen Zahnraddicken werden durch Distanzring als Platzhalter ausgeglichen. Dafür ist aber ein hoher manueller Montageaufwand notwendig. Es ist also bekannt, dass unterschiedliche Pumpenvarianten einen Kostenfaktor darstellen.

### Stand der Technik

Aus der DE 10 2009 047 619 A1 ist eine Zahnradpumpe mit einem Gehäusering bekannt, in dem die Zahnräder angeordnet sind. Der Gehäusering kann für verschiedene Pumpenversionen mit unterschiedlichen Förderleistungen und Zahnraddicken verwendet werden. Für die Pumpenversion mit einer kleineren Förderleistung kann ein dünnerer Gehäusering verwendet werden, der keine Durchbrüche und Kanäle aufweist und daher kostengünstiger zu fertigen ist. Die einzelnen Gehäuseringe für die jeweilige Pumpenversion werden aber einzeln durch ein spanendes Fertigungsverfahren gefertigt.

Die CN 1 06 870 360 A offenbart ein Gehäuse für eine Zahnradpumpe, das aus einem Metall-Kunststoff-Verbundwerkstoff besteht und durch Spritzgießen gefertigt wird. Dabei ist eine Variation der Geometrie des Gehäuses mit hohen Werkzeug- und Umrüstkosten verbunden.

Aus der WO 2012 / 066 026 A2 ist eine Drehkolbenpumpe mit einem Pumpengehäuse bekannt, das aus zwei Gehäuseschalen zusammengesetzt ist. Das Pumpengehäuse wird dabei aus zwei halbkreisförmigen Gehäusehalbschalen gefertigt, die durch Auftrennen eines kreisrunden Gehäuses hergestellt werden. Das kreisförmige Gehäuse wird dabei gegossen und spanend nachbearbeitet. Um Verformung der Gehäusehalbschalen beim Trennen durch Eigenspannungen zu verhindern, wird das kreisförmige Gehäuse doppelwandig ausgebildet.

Die DE 10 2019 102 073 A1 und die US 3 986 797 A zeigen jeweils Zahnradpumpen mit einem Gehäuse.

### Zusammenfassung der Offenbarung

Die Aufgabe der Offenbarung besteht deshalb darin, einen Universal-Pumpengehäuserohling oder Universal-Pumpengehäuseabschnittrohling herzustellen, der mit minimalem Aufwand und kostengünstig zu einem Pumpengehäuse oder Pumpengehäuseabschnitt für Pumpen unterschiedlicher Leistung weiterverarbeiten werden kann.

Diese Aufgabe wird gemäß der vorliegenden Offenbarung durch ein Verfahren zur Herstellung einer Pumpe zur Verwendung in einer Blutbehandlungseinrichtung, vorzugsweise einer Dialysemaschine mit den Merkmalen des Anspruchs 1 gelöst. Ferner wird die Aufgabe durch eine Pumpe gemäß Anspruch 9 und eine Dialysemaschine gemäß Anspruch 14 gelöst. Vorteilhafte Weiterbildungen der Offenbarung sind Gegenstand der beigefügten Unteransprüche.

Die vorliegende Offenbarung betrifft ein Verfahren zum Herstellen von Pumpen unterschiedlicher Leistung zur Verwendung in einer Blutbehandlungsvorrichtung, vorzugsweise einer Dialysemaschine. Die Pumpe weist ein Pumpengehäuse oder einen Pumpengehäuseabschnitt zur lagernden Aufnahme einer Fluidfördereinrichtung, vorzugsweise zweier in Kämmeingriff befindlicher Zahnräder, auf. Das offenbarungsgemäße Verfahren weist die folgenden Schritte auf: Urformen eines Universal-Pumpengehäuserohlings oder Universal-Pumpengehäuseabschnittrohlings mit allen, den unterschiedlichen Pumpen gemeinsamen Gehäusemerkmalen sowie mit solchen Übermaßen, die eine spanabhebende Bearbeitung zur Herstellung aller unterschiedlichen Pumpen aus demselben urgeformten Universal-Pumpengehäuserohling oder Universal-Pumpengehäuseabschnittrohling erlauben; Individualisieren des Universal-Pumpengehäuserohlings oder Universal-Pumpengehäuseabschnittrohlings durch spanabhebende Bearbeitung im Bereich der Übermaße zur Erreichung der jeweiligen Leistung.

Die Gehäusemerkmale sind dabei beispielsweise eine Tiefe einer Aufnahmetasche für die Fluidfördereinrichtung, ein Durchmesser der Aufnahmetasche, eine Ausgestaltung von Zahnrad-Aufnahmeflächen, Schlauchtüllen und die Dichtflächen. Die Gehäusemerkmale werden bei der Herstellung des Pumpengehäuses oder Pumpengehäuseabschnitts aus dem Universal-Pumpengehäuserohling oder Universal-Pumpengehäuseabschnittrohling vorzugsweise spanend bearbeitet.

Unter den Übermaßen des Universal-Pumpengehäuserohlings oder Universal-Pumpengehäuseabschnittrohlings ist in diesem Zusammenhang zu verstehen, dass der Universal-Pumpengehäuserohling oder Universal-Pumpengehäuseabschnittrohling größer ist als das Pumpengehäuse oder der Pumpengehäuseabschnitt. Die Übermaße werden bei der Fertigung des Pumpengehäuseabschnitts aus dem Universal-Pumpengehäuseabschnittrohling spanend abgetragen. Beispielsweise beträgt die Tiefe der Zahntaschen 9,5 mm bzw. 10,5 mm. Bei dem Universal-Pumpengehäuseabschnittrohling ist die Tiefe der Zahntaschen weniger als 9,5 mm. So wird sichergestellt, dass ausreichend Material vorhanden ist, um die kleinste Tiefe spanend zu fertigen, beispielsweise zu fräsen. Genauso ist hat die Aufnahmetasche des Pumpengehäuses oder Pumpengehäuseabschnitts einen Durchmesser von 12,7 mm. Dieser Durchmesser ist bei dem Universal-Pumpengehäuserohling oder Universal-Pumpengehäuseabschnittrohling geringer und wird nachgearbeitet. Die Wandstärke des Universal-Pumpengehäuserohlings oder Universal-Pumpengehäuseabschnittrohlings ist mit ca. 2,5 mm größer als die Wandstärke des Pumpengehäuses oder Pumpengehäuseabschnitts. Ferner weisen die Schlauchtüllen einen Innendurchmesser von ca. 5 mm auf. Der Universal-Pumpengehäuserohling oder Universal-Pumpengehäuseabschnittrohling weist ferner 2° Ausformschrägen auf.

In anderen Worten wird der Universal-Pumpengehäuserohling oder Universal-Pumpengehäuseabschnittrohling durch Urformen hergestellt. Der Universal-Pumpengehäuserohling oder Universal-Pumpengehäuseabschnittrohling weist alle Merkmale auf, die den unterschiedlichen Pumpen gemeinsam sind. Ferner hat der Universal-Pumpengehäuserohling oder Universal-Pumpengehäuseabschnittrohling ein solches Übermaß, dass aus dem Universal-Pumpengehäuserohling alle unterschiedlichen Pumpen spanend gefertigt werden können. Eine spanende Fertigungstechnik kann dabei, aber nicht ausschließlich, das Fräsen sein. Im Sinne der Offenbarung kann aber auch jede andere spanende Bearbeitung durchgeführt werden. In einem weiteren Schritt wird der Universal-Pumpengehäuserohling oder Universal-Pumpengehäuseabschnittrohling individualisiert. Dabei werden insbesondere das Pumpengehäuse bzw. der Pumpengehäuseabschnitt durch spanende Bearbeitung in eine solche Dimensionierung gebracht, dass die Pumpe die geeigneten Fluidfördereinrichtung aufnehmen kann, die die geforderte Leistung der Pumpe bereitstellen.

Der Universal-Pumpengehäuserohling oder Universal-Pumpengehäuseabschnittrohling wird durch Urformen gefertigt. Beispielhaft aber nicht ausschließlich werden als mögliche Fertigungstechniken Druckguss oder Metallpulverspritzgießen genannt. Es ist aber auch eine Fertigung durch andere urformende Fertigungstechniken möglich.

Es ist anzumerken, dass der Schritt des Individualisierens nicht solche spanenden Bearbeitungsschritte umfasst, die bei urgeformten und insbesondere gegossenen standardmäßig durchgeführt werden. Zu solchen Bearbeitungsschritten zählen insbesondere Entgraten oder Nachbearbeitungen von Dichtflächen, Passungen oder ähnlichen Flächen, die nicht durch Urformen in genügender Qualität hergestellt werden können.

Die Leistung der Pumpen ist von der Dimensionierung der Pumpen abhängig. Dabei hat in erster Linie die Dimensionierung der jeweiligen Fluidfördereinrichtung Einfluss auf die Leistung der Pumpen. Abhängig davon muss auch die Dimensionierung des Pumpengehäuses bzw. des Pumpengehäuseabschnitts, der die Fluidfördereinrichtung aufnimmt, entsprechend dimensioniert sein. Deshalb betrifft die vorliegende Offenbarung in anderen Worten ein Verfahren zum Herstellen eines Pumpengehäuses einer Mehrzahl unterschiedlich geformter und/oder dimensionierter Pumpen einer Blutbehandlungsvorrichtung, vorzugsweise einer Dialysemaschine. Der Pumpengehäuseabschnitt nimmt die Fluidfördereinrichtung, vorzugsweise zwei in Kämmeingriff befindliche Zahnräder, lagernd auf. Das Verfahren weist die folgenden Schritte auf: Urformen eines Universal-Pumpengehäuserohlings oder Universal-Pumpengehäuseabschnittrohlings mit allen, den unterschiedlichen Pumpen gemeinsamen Merkmalen sowie mit solchen Übermaßen, die eine spanabhebende Bearbeitung zur Herstellung aller unterschiedlichen Pumpen aus demselben urgeformten Pumpengehäuserohling erlauben. Individualisieren des Universal-Pumpengehäuserohlings oder Universal-Pumpengehäuseabschnittrohlings durch spanabhebende Bearbeitung zu dessen Anpassung an die individuellen Konstruktionsmerkmale und/oder Dimensionierung der jeweiligen Pumpe zur Erreichung unterschiedlicher Leistungsspezifikationen.

Sollte es sich bei der Pumpe um eine Zahnradpumpe handeln, so umfasst die Fluidfördereinrichtung zwei miteinander kämmende Zahnräder. Der Pumpengehäuseabschnitt ist dann eine Zahnradaufnahme in der die Zahnräder drehbar gelagert sind. Durch eine Variation einer Zahnraddicke kann die Leistung der Zahnradpumpe eingestellt werden. In dem vorliegenden Fall wird also eine Zahnradaufnahme, die die Zahnräder aufnimmt, derart tief ausgefräst, dass sie genau die Zahnraddicke aufnehmen kann, die für die jeweilige Pumpenleistung notwendig ist.

Das offenbarungsgemäße Verfahren zum Herstellen der Pumpen mit unterschiedlicher Leistung weist die folgenden Vorteile auf. Der Universal-Pumpengehäuserohling oder Universal-Pumpengehäuseabschnittrohlings kann durch wenige Bearbeitungsschritte in das Pumpengehäuse oder Pumpengehäuseabschnitt für die Pumpen unterschiedlicher Leistung weiterverarbeitet werden. Insbesondere die Dimensionierung der Aufnahmetasche für unterschiedlich dicke Zahnräder ist mit wenigen Bearbeitungsschritten möglich. Das wiederum ermöglicht es, die Pumpen unterschiedlicher Leistung kostengünstig herzustellen. Da der Universal-Pumpengehäuserohling oder Universal-Pumpengehäuseabschnittrohlings für alle Pumpenvariationen benutzt werden kann, kann er in großen Mengen hergestellt werden. Das senkt die Fertigungskosten pro Stück. Alle anderen Bauteile der Pumpe sind gleich und können so in großen Mengen bereitgestellt werden. Ferner wird das Pumpengehäuse oder der Pumpengehäuseabschnitt im Gegensatz zur herkömmlichen Herstellung nicht gefräst. Durch die Herstellung durch Urformen fällt deutlich weniger Abfall an und Material kann eingespart werden. Durch die urformende Fertigung kann also insbesondere der Materialverbrauch/ Ausschuss im Vergleich zur spanenden Fertigung vermindert werden. Durch Urformen können dünnere Wandstärken des Pumpengehäuseabschnitts erreicht werden, was den Materialverbrauch noch weiter senkt. Des Weiteren können werkzeugfallende Universal-Pumpengehäuserohlinge hergestellt werden, die keine aufwändige Nachbearbeitung benötigen. So können Montageschritte und damit Kosten gespart werden. Besonders das Anpassen des Pumpengehäuseabschnitts an die unterschiedliche Zahnraddicke durch unterschiedlich dicke Dichtplatten entfällt gegenüber dem herkömmlichen Herstellprozess. Durch das offenbarungsgemäße Herstellverfahren können also kostengünstig unterschiedliche Pumpen, insbesondere eine Entgasungspumpe, eine Dialysierflüssigkeit-Eingangspumpe und eine Dialysierflüssigkeit-Ausgangspumpe, hergestellt werden.

Zusammenfassend liegt der Kern der Offenbarung darin, dass ein allgemeiner Universal-Pumpengehäuserohling oder Universal-Pumpengehäuseabschnittrohlings für eine Pumpe durch Urformen hergestellt wird, der in unterschiedliche Pumpengehäuseabschnitte weiterverarbeitet wird, die genau so dimensioniert sind, dass sie Fluidfördereinheiten aufnehmen können, die die von der Pumpe geforderte Leistung bereitstellen.

Nach einem weiteren optionalen Merkmal der Offenbarung werden beim Individualisieren des Universal-Pumpengehäuserohlings Aufnahmetaschen für die Fluidfördereinrichtung derart ausgebildet, insbesondere durch spanende Bearbeitung vergrößert oder verkleinert, dass jene, entsprechende Abmessungen aufweisende Fluidfördereinrichtung aufnehmbar ist, die die jeweilig geforderte Leistung bereitstellt.

Wie vorstehend beschrieben, ist die Leistung der Pumpe von der Dimensionierung der Fluidfördereinrichtung abhängig. Die Dimensionierung des Pumpengehäuseabschnitts muss an die jeweilige Fluidfördereinrichtung angepasst sein. Darum werden bei dem Individualisieren des Universal-Pumpengehäuserohlings oder Universal-Pumpengehäuseabschnittrohlings Aufnahmetaschen derart vergrößert oder verkleinert, dass die Aufnahmetaschen genau die Fluidfördereinrichtung aufnehmen, die an die geforderte Leistung angepasst ist. Das Vergrößern oder Verkleinern der Aufnahmetaschen wird dabei durch spanende Bearbeitung, insbesondere Fräsen, realisiert. Dadurch können Pumpen mit unterschiedlicher Leistung gefertigt werden, indem lediglich die Dimensionierung der Aufnahmetasche des Pumpengehäuseabschnitts angepasst wird. Alle anderen Bauteile der Pumpe und die Dimensionierung des Pumpengehäuseabschnitts sind bei den Pumpen unterschiedlicher Leistung gleich.

Nach einem weiteren optionalen Merkmal der Offenbarung werden beim Individualisieren des Universal-Pumpengehäuserohlings oder Universal-Pumpengehäuseabschnittrohlings Zahnrad-Auflageflächen der Aufnahmetaschen spanend axial abgetragen werden, um eine Tiefe der Aufnahmetaschen zu vergrößern. Für Pumpen, bei denen eine hohe Leistung gefordert ist, müssen die Zahnräder entsprechend dick sein. Für die dicken Zahnräder müssen die Aufnahmetaschen entsprechend groß sein. Damit die Aufnahmetaschen die Zahnräder aufnehmen können, werden die Zahnrad-Auflageflächen derart abgefräst, dass die Aufnahmetaschen tiefer sind und auch dicke Zahnräder aufnehmen können. Durch das Abfräsen der Zahnrad-Auflageflächen kann der Universal-Pumpengehäuserohling oder Universal-Pumpengehäuseabschnittrohling mit geringem Aufwand in ein Pumpengehäuse oder einen Pumpengehäuseabschnitt für eine Pumpe mit hoher Leistung verarbeitet werden. Alternativ oder ergänzend hierzu können auch die Durchmesser der Aufnahmetaschen spanend vergrößert werden, um so z.B. Zahnräder mit größeren Durchmessern einsetzen zu können.

Die Größe der Aufnahmetaschen wird durch den Abstand von einem Anschlussflansch des Pumpengehäuses oder Pumpengehäuseabschnitts zu den Zahnrad-Auflageflächen definiert. Der Anschlussflansch definiert die Fläche, über die die Zahnräder nicht herausstehen dürfen. Die Zahnrad-Auflagefläche ist die Fläche, auf der sich die Zahnräder in den Aufnahmetaschen drehen.

Nach einem weiteren optionalen Merkmal der Offenbarung weist eine axiale Erstreckung des Universal-Pumpengehäuserohlings oder Universal-Pumpengehäuseabschnittrohlings ein Übermaß auf. D.h. der Universal-Pumpengehäuserohling oder Universal-Pumpengehäuseabschnittrohlings hat eine längere axiale Erstreckung als das Pumpengehäuse oder Pumpengehäuseabschnitt und wird zur Verkleinerung der Aufnahmetasche axial spanend gekürzt.

In anderen Worten hat der Universal-Pumpengehäuserohling oder Universal-Pumpengehäuseabschnittrohling mehr Material als jeder der Pumpengehäuseabschnitte, in die der Universal-Pumpengehäuserohling oder Universal-Pumpengehäuseabschnittrohling weiterverarbeitet wird. Deshalb kann aus dem Universal-Pumpengehäuserohling durch spanende Bearbeitung jeder der unterschiedlichen Pumpengehäuseabschnitte gefertigt werden. Unter die axiale Erstreckung des Universal-Pumpengehäuserohlings oder Universal-Pumpengehäuseabschnittrohlings fällt auch ein Anschlussflansch, der an einer Seite des Pumpengehäuseabschnitts ausgebildet ist. Der Anschlussflansch kann abgefräst werden, um die Aufnahmetasche für die Zahnräder zu verkleinern. Der Abstand zwischen der Zahnrad-Auflagefläche und dem Anschlussflansch begrenzt die Dicke der Zahnräder, die zwischen diesen Flächen aufgenommen werden können. Wird der Anschlussflansch abgefräst, so verkleinert sich dieser Abstand.

Als Anschlussflansch ist die Stirnseite des Universal-Pumpengehäuserohlings oder Universal-Pumpengehäuseabschnittrohlings definiert, die in Richtung der Zahnräder zeigt und sowohl eine Fläche zur Aufnahme einer Dichtplatte, als auch eine Fläche zum Verbinden mit einem Antriebsgehäuse der Pumpe umfasst. Die axiale Erstreckung soll als eine Erstreckung in Richtung einer Antriebswelle der Pumpe verstanden werden.

Nach einem weiteren optionalen Merkmal der Offenbarung werden beim Individualisieren des Universal-Pumpengehäuserohlings oder Universal-Pumpengehäuseabschnittrohlings die Innenseiten der Aufnahmetaschen spanend abgetragen, um die Durchmesser der Aufnahmetaschen zu vergrößern. Es ist möglich, die Zahnrad-Auflagefläche abzufräsen, um die Tiefe der Aufnahmetasche zu vergrößern und durch dickere Zahnräder eine höhere Leistung der Pumpe zu erreichen. Es ist ebenfalls möglich, die Innenseiten der Aufnahmetaschen radial abzufräsen, um den Durchmesser der Aufnahmetaschen zu vergrößern. Dadurch können Zahnräder mit einem größeren Durchmesser in der Pumpe eingesetzt werden und durch den größeren Zahnraddurchmesser die Leistung der Pumpe erhöht werden.

Nach einem weiteren optionalen Merkmal der Offenbarung werden beim Urformen des Universal-Pumpengehäuserohlings Schlauchtüllen geformt, die integral mit dem Universal-Pumpengehäuserohling ausgebildet sind. Die Schlauchtüllen sind dazu ausgebildet und vorbereitet, fluidführende Schläuche daran anzuschließen. Die Schlauchtüllen können als ein integraler Teil des Universal-Pumpengehäuserohlings urgeformt werden. Bei der herkömmlichen spanenden Fertigung der einzelnen Pumpengehäuseabschnitte müssen die Schlauchtüllen nachträglich manuell montiert werden. Diese Montage entfällt durch das offenbarungsgemäße Verfahren. Dadurch lassen sich Arbeitszeit bei der Montage und damit Kosten einsparen.

Nach einem weiteren optionalen Merkmal der Offenbarung wird beim Individualisieren des Universal-Pumpengehäuserohlings ein Durchgangskanal von Zahnrad-Auflageflächen der Aufnahmetasche zu einem Anschlussstutzen zum Anbringen eines Rückschlagventils aufgebohrt. Die Dialysierflüssigkeit-Eingangspumpe weist ein Rückschlagventil auf, das bei Überdruck öffnet und die Dialysemaschine vor zu hohen Drücken schützt. Wenn ein Druck in der Dialysemaschine zu hoch ist, kann über das Ventil Druck abgelassen werden. Das Rückschlagventil wird an Befestigungsstutzen befestigt, die aus dem Universal-Pumpengehäuserohling hervorstehen. Das Rückschlagventil ist mit Anschlussstutzen des Universal-Pumpengehäuserohlings verbunden. Damit eine fluidische Verbindung zwischen dem Inneren der Pumpe und dem Rückschlagventil hergestellt wird, wird der Durchgangskanal von den Zahnrad-Auflageflächen der Aufnahmetasche und dem Anschlussstutzen aufgebohrt. Die Anschlussstutzen für das Rückschlagventil sind in dem Universal-Pumpengehäuserohling ausgebildet. Bei Pumpen, die nicht die Dialysierflüssigkeit-Eingangspumpe sind, wird der Durchgangskanal nicht aufgebohrt und damit keine Fluidverbindung zwischen der Aufnahmetasche bzw. dem Zahnradraum und dem Äußeren hergestellt.

Durch das Aufbohren des Durchgangskanals kann kostengünstig eine weitere Pumpenvariation, nämlich die Dialysierflüssigkeit-Eingangspumpe hergestellt werden. Um aus dem Universal-Pumpengehäuserohling einen Pumpengehäuseabschnitt für die Dialysierflüssigkeit-Eingangspumpe herzustellen, werden die Zahnrad-Auflageflächen der Aufnahmetasche gefräst und der Durchgangskanal aufgebohrt.

Nach einem weiteren optionalen Merkmal der Offenbarung werden der Anschlussflansch und ein Montageflansch des Antriebsgehäuses ausschließlich unter Zwischenfügen von Dichtungen ohne zusätzliche Distanzringe zur individuellen Einstellung der axialen Abmessung der Aufnahmetaschen miteinander verbunden. Der Anschlussflansch und der Montageflansch werden dabei zusammengeschraubt. Dafür weist der Anschlussflansch Gewinde auf. Zwischen die beiden Flansche wird zusätzlich ein Spalttopf verschraubt, der die Zahnräder axial abdichtet. Ferner liegt an dem Anschlussflansch eine Dichtplatte an, die die Zahnräder ebenfalls axial gegenüber der Magnetkupplung abdichtet. Diese Dichtungen sind die einzigen Bauteile, die zwischen dem Anschlussflansch und dem Montageflansch angeordnet sind. Herkömmlich werden die unterschiedlichen Zahnraddicken unterschiedlicher Pumpen durch unterschiedlich dicke Dichtplatten oder Distanzringe ausgeglichen. Da die Aufnahmetaschen des Pumpengehäuses oder Pumpengehäuseabschnitts der vorliegenden Offenbarung die unterschiedlichen Zahnraddicken ausgleichen, ist der Einsatz von zusätzlichen Distanzringen oder unterschiedlich dicken Dichtplatten nicht notwendig.

Die Aufgabe der vorliegenden Offenbarung wird ferner durch eine Pumpe, vorzugsweise eine Zahnradpumpe, zur Verwendung in einer Blutbehandlungsvorrichtung, vorzugsweise einer Dialysemaschine, gelöst. Die Pumpe weist einen elektrischen Antrieb, insbesondere einen Elektromotor mit einem auf einer Antriebswelle montierten oder ausgebildeten Rotor auf, der in einem Antriebsgehäuse gelagert ist, welches einen Montageflansch hat. Ferner hat die Pumpe ein Pumpengehäuse oder Pumpengehäuseabschnitt mit einem Anschlussflansch. Das Pumpengehäuse oder Pumpengehäuseabschnitt ist nach dem offenbarungsgemäßen Verfahren durch Individualisierung des Universal-Pumpengehäuserohlings oder Universal-Pumpengehäuseabschnittrohlings hergestellt und mit dem Anschlussflansch an den Montageflansch angeflanscht. Der Anschlussflansch wiederum ist derart an dem Montageflansch befestigt, dass die Antriebswelle in einen von den Aufnahmetaschen gemeinsam ausgebildeten Innenraum des Pumpengehäuses oder Pumpengehäuseabschnitts ragt. Ferner weist die Pumpe die Fluidfördereinrichtung in Form zweier in Kämmeingriff befindlicher Zahnräder auf, die in Wirkeingriff mit der Antriebswelle stehen und der Innenseite der Aufnahmetaschen abgleiten.

In anderen Worten treibt der Elektromotor die Zahnräder der Pumpe direkt oder unter Umständen über entsprechende Kupplungen oder ein Getriebe an. Die Zahnräder sind in dem Pumpengehäuse oder Pumpengehäuseabschnitt in der Aufnahmetasche aufgenommen. Das Pumpengehäuse oder der Pumpengehäuseabschnitt wird nach dem offenbarungsgemäßen Verfahren aus dem Universal-Pumpengehäuserohling oder Universal-Pumpengehäuseabschnittrohling hergestellt. Die Aufnahmetasche ist dabei derart dimensioniert, dass die Zahnräder in die Aufnahmetaschen hineinpassen.

Das offenbarungsgemäße Verfahren beinhaltet dabei das Urformen des Universal-Pumpengehäuserohlings und das anschließende Individualisieren des urgeformten Universal-Pumpengehäuserohlings zu unterschiedlichen Pumpengehäuseabschnitten. Da das Individualisieren nur wenige Fertigungsschritte benötigt, können Montage- und Bearbeitungskosten gespart werden. Dadurch können die Pumpen unterschiedlicher Leistung kostengünstig aus nur einem einzigen Universal-Pumpengehäuserohling gefertigt werden. Aus dem Universal-Pumpengehäuserohling können mit einer geringen Anzahl an Fertigungsschritten die unterschiedlichen Pumpengehäuseabschnitte der jeweiligen Pumpen gefertigt werden. Dabei ist insbesondere der Materialverbrauch im Vergleich zur spanenden Fertigung reduziert. Dadurch, dass nur wenige Fertigungsschritte zur (spanenden) Nachbearbeitung des Gussteils/ bzw. des gegossenen Universal-Pumpengehäuserohlings notwendig sind, können Arbeitszeit und damit Kosten gespart werden. Die unterschiedlichen Pumpen unterscheiden sich untereinander zum einen in der Leistung und zum anderen in anderen Aspekten, wie dem Vorhandensein eines Rückschlagventils. Wie vorstehend erläutert, werden die unterschiedlichen Pumpengehäuseabschnitte aus dem gleichen Universal-Pumpengehäuserohling gefertigt. Die restlichen Komponenten der unterschiedlichen Pumpen sind alle gleich und können somit ebenfalls in großen Stückzahlen günstig hergesetzt werden. Somit können die unterschiedlichen Pumpen kostengünstig aus den gleichen Bauteilen hergestellt werden.

Weiter sind der Anschlussflansch und der Montageflansch ausschließlich unter Zwischenfügen von Dichtungen ohne zusätzliche Distanzringe zur individuellen Einstellung der axialen Abmessung der Aufnahmetaschen miteinander verbunden. Der Anschlussflansch und der Montageflansch werden derart verschraubt, dass der Spalttopf zwischen den beiden Flanschen verschraubt ist. Die Zahnräder der Pumpe werden durch die Dichtplatte und den Spalttopf axial abgedichtet. Beim Verbinden des Anschlussflansches und des Montageflansches werden keine zusätzlichen Dichtplatten oder Distanzringe benötigt, die durch ihre axialen Abmessungen die axialen Abmessungen der Zahnräder ausgleichen. Die unterschiedlichen Zahnraddicken werden offenbarungsgemäß durch die Dimensionierung des Pumpengehäuses oder Pumpengehäuseanschnitts ausgeglichen.

Nach einem weiteren optionalen Merkmal der Offenbarung sind die Zahnräder auf Zylinderstiften gelagert, die in Aufnahmebohrungen des Pumpengehäuses oder Pumpengehäuseabschnitts verpresst sind. Dadurch müssen keine zusätzlichen Lager verbaut werden.

Nach einem weiteren optionalen Merkmal der Offenbarung sind die Zahnräder auf Gleitlagern in dem Pumpengehäuseabschnitt gelagert. Durch den Einsatz der Gleitlager wird eine Reibung zwischen den Zahnrädern und dem Pumpengehäuseabschnitt reduziert.

Nach einem weiteren optionalen Merkmal der Offenbarung sind die Zahnräder auf gleitenden Vollwellen in dem Pumpengehäuseabschnitt gelagert. Dadurch müssen keine zusätzlichen Lager für die Zahnräder eingebaut werden. Das spart einen zusätzlichen Fertigungsschritt und damit Kosten und Arbeitsaufwand.

Nach einem weiteren optionalen Merkmal der Offenbarung weist der Pumpengehäuseabschnitt eine gussgerechte Wanddicke auf. Die Wanddicke des Pumpengehäuseabschnitts ist geringer als die eines herkömmlich spanend gefertigten Pumpengehäuseabschnitts. Das ist zum einen vorteilhaft, weil so beim Gießen bzw. Urformen keine Lunker entstehen. Zum anderen verbessert sich durch die geringe Wandstärke das thermische Verhalten der Zahnradpumpe. Da die Zahnräder aus einem Kunststoff gefertigt sind, weisen sie einen anderen Wärmeausdehnungskoeffizienten als der metallene Pumpengehäuseabschnitt auf. Bei einer Erwärmung dehnen sich die Zahnräder schneller als der Pumpengehäuseabschnitt aus und können dadurch verklemmen. Dies wird durch die möglichst dünne Wandstärke des Pumpengehäuseabschnitts vermindert, da sich die geringere Masse schneller erwärmt und dadurch schneller ausdehnt. Dadurch kann verhindert werden, dass sich die Zahnräder, die aus einem Kunststoff wie beispielsweise PEEK gefertigt sind, aufgrund ihrer höheren Wärmeausdehnungskoeffizienten schneller ausdehnen als der Pumpengehäuseabschnitt und dadurch verklemmen. Als Folge kann die Toleranz zwischen der Innenseite des Pumpengehäuseabschnitts und dem jeweiligen Zahnrad enger gesetzt werden, was zu einer besseren Pumpenleistung bzw. Fördermenge der Zahnradpumpe führt.

Als gussgerechte Wanddicke ist in diesem Zusammenhang eine Wand ohne Materialanhäufungen zu verstehen. Die Wand weist auch keine abrupten Änderungen der Wandstärke auf. Durch die fehlenden Materialanhäufungen wird die Wandstärke geringer. Dadurch vermindert sich auch ein möglicher thermischer Verzug der Wand des Pumpengehäuseabschnitts.

Es ist ebenfalls denkbar, dass der Pumpengehäuseabschnitt auch aus einem Kunststoff, beispielsweise PEEK (Polyetheretherketon) oder POM (Polyoxymethylene) gefertigt ist.

Die vorliegende Offenbarung betrifft ferner eine Dialysemaschine zum extrakorporalen Reinigen von Blut mit mehreren Pumpen unterschiedlicher Leistung, vorzugsweise Zahnradpumpen, die nach dem offenbarungsgemäßen Verfahren hergestellt sind. Die Dialysemaschine weist mehrere Zahnradpumpen auf, die zum Entgasen und Fördern von Flüssigkeiten vorgesehen und ausgebildet sind. Dabei handelt es sich um eine Entgasungspumpe zum Entgasen einer Flüssigkeit, insbesondere Dialysierflüssigkeit. Bei den Zahnradpumpen handelt es sich ferner um eine Dialysierflüssigkeit-Eingangspumpe und eine Dialysierflüssigkeit-Ausgangspumpe zur Bilanzierung der Dialysierflüssigkeit.

Die unterschiedlichen Zahnradpumpen haben unterschiedliche Funktionen und unterschiedliche Anforderungen. So unterscheidet sich beispielsweise die geforderte Flussmenge/ der Durchfluss/ der Volumenstrom der Zahnradpumpen. Die Entgasungspumpe muss einen höheren Volumenstrom liefern als die anderen Zahnradpumpen und weist deshalb eine größere Zahnraddicke auf. Die Dialysierflüssigkeit-Eingangspumpe weist ein Rückschlagventil auf, um die Dialysemaschine vor zu hohen Drücken zu schützen. Durch diese Unterschiede weisen die jeweiligen Zahnradpumpen unterschiedlich ausgestaltete/ konstruierte Pumpengehäuseabschnitte auf. Beispielsweise ist der Pumpengehäuseabschnitt der Entgasungspumpe tiefer als die Pumpengehäuseabschnitte der anderen Pumpen, um die dickeren Zahnräder der Entgasungspumpe aufzunehmen. Diese unterschiedlich gestalteten Pumpengehäuseabschnitte sind aber trotzdem aus demselben/ dem gleichen Universal-Pumpengehäuserohling gefertigt. Der Universal-Pumpengehäuserohling wird durch Urformen werkzeugfallend gefertigt und muss nach dem Urformen nur minimal spanend bearbeitet werden. Die Aufnahmetasche einer jeweiligen Pumpe ist genauso dimensioniert, dass die Zahnräder aufgenommen werden bzw. in die Aufnahmetasche abschließend passen, die die geforderte Leistung liefern.

Zusammenfassend weist die vorliegende Offenbarung die folgenden Vorteile auf:
- Günstig Herstellbar
- Modulares Baukastensystem
- Effizienter Materialeinsatz
- Keine Montage der Schlauchtüllen notwendig
- Weniger Neigung zum Zahnradklemmen bei Temperaturwechseln auf Grund der geringeren Wandstärken
- Geringeres Gewicht
- Platzsparend

### Kurzbeschreibung der Figuren

Fig. 1 zeigt eine offenbarungsgemäße Pumpe nach einer ersten Ausführungsform;
Fig. 2 zeigt einen Längsschnitt durch die offenbarungsgemäße Pumpe in Fig. 1;
Fig. 3a zeigt eine isometrische Darstellung eines Universal-Pumpengehäuserohlings;
Fig. 3b zeigt eine Schnittansicht des Universal-Pumpengehäuserohlings in Fig. 3a;
Fig. 3c zeigt eine Schnittansicht eines Pumpengehäuseabschnitts für eine Entgasungspumpe nach der ersten Ausführungsform;
Fig. 3d zeigt eine Schnittansicht eines Pumpengehäuseabschnitts für eine Dialysierflüssigkeit-Eingangspumpe nach einer zweiten Ausführungsform;
Fig. 3e zeigt eine Schnittansicht eines Pumpengehäuseabschnitts für eine Dialysierflüssigkeit-Ausgangspumpe nach einer dritten Ausführungsform;
Fig. 4 zeigt eine Schnittansicht durch eine offenbarungsgemäße Pumpe nach einer vierten Ausführungsform;
Fig. 5 zeigt eine schematische Ansicht einer offenbarungsgemäßen Dialysemaschine; und
Fig. 6 zeigt eine schematische Ansicht zweier sich in Kämmeingriff befindlicher Zahnräder.

### Beschreibung der Ausführungsformen

Nachstehend werden Ausführungsformen der vorliegenden Offenbarung auf der Basis der zugehörigen Figuren beschrieben.

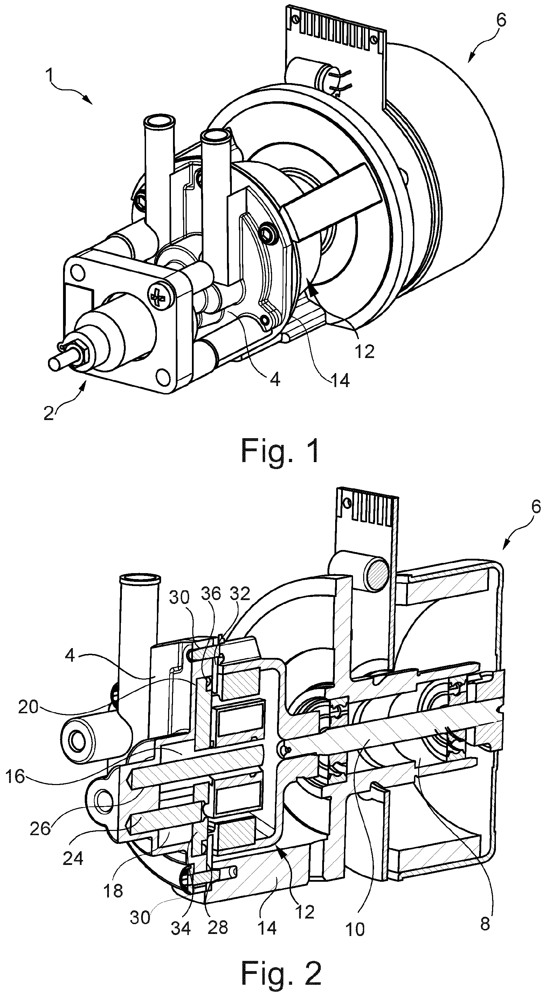

Fig. 1 zeigt eine Pumpe 1 nach einer ersten Ausführungsform. Die Pumpe 1 nach der ersten Ausführungsform ist insbesondere eine Dialysierflüssigkeit-Eingangspumpe für eine Blutbehandlungsvorrichtung, vorzugsweise eine Dialysemaschine. Die Dialysierflüssigkeit-Eingangspumpe unterscheidet sich durch ein Rückschlagventil 2, das an einem Pumpengehäuse oder Pumpengehäuseabschnitt 4 der Pumpe 1 angeschraubt ist, von anderen Pumpen 1. Fig. 2 zeigt einen Längsschnitt durch eine offenbarungsgemäße Pumpe 1 ohne das Rückschlagventil 2. Die Pumpe 1 weist einen elektrischen Antrieb 6, vorzugsweise einen Elektromotor auf. Der elektrische Antrieb 6 hat einen Rotor 8, der auf einer Antriebswelle 10 montiert ist. Der elektrische Antrieb 6 ist über die Antriebswelle 10 mit einer Magnetkupplung 12 verbunden. Die Magnetkupplung 12 ist innerhalb eines Antriebsgehäuses 14 angebracht. Durch einen magnetischen Kraftschluss der Magnetkupplung 12 wird die Antriebswelle 10 in einen Wirkeingriff mit einem ersten Zahnrad 16 gebracht. Das erste Zahnrad 16 kämmt mit einem zweiten Zahnrad 18 und treibt dieses an. Die zwei Zahnräder 16, 18 sind in dem Pumpengehäuse oder Pumpengehäuseabschnitt 4 drehbar gelagert. Der Pumpengehäuseabschnitt 4 weist eine im Wesentlichen runde Grundplatte 19 mit einem Anschlussflansch 20 auf. Zur Aufnahme der Zahnräder 16, 18 bildet der Pumpengehäuseabschnitt 4 in der Grundplatte 19 eine (ovale) Aufnahmetasche 22 aus. Die zwei Zahnräder 16, 18 sind jeweils auf einem Zylinderstift 24 drehbar gelagert, die in Aufnahmebohrungen 26 des Pumpengehäuses oder Pumpengehäuseabschnitts 4 verpresst sind. Der Pumpengehäuseabschnitt 4 wird nachfolgend detailliert beschrieben. Der Anschlussflansch 20 des Pumpengehäuseabschnitts 4 ist mit einem Montageflansch 28 des Antriebsgehäuse 14 verschraubt. Dazu weist der Anschlussflansch 20 Gewinde 30 auf, die dazu vorbereitet sind, Schrauben aufzunehmen, mit denen der Anschlussflansch 20 und der Montageflansch 28 miteinander verschraubt sind. Der Pumpengehäuseabschnitt 4 und das Antriebgehäuse 14 sind mit einem Spalttopf 32 abgedichtet. Der Spalttopf 32 wird durch die Gewinde 30 zwischen dem Pumpengehäuseabschnitt 4 und dem Antriebgehäuse 14 verschraubt. Die beiden Zahnräder 16, 18 sind gegenüber der Magnetkupplung 12 und dem Inneren des Antriebgehäuse 14 mit einer Dichtplatte 34 abgedichtet. Die Pumpe 1 wird mit einem Dichtring 36 nach außen abgedichtet.

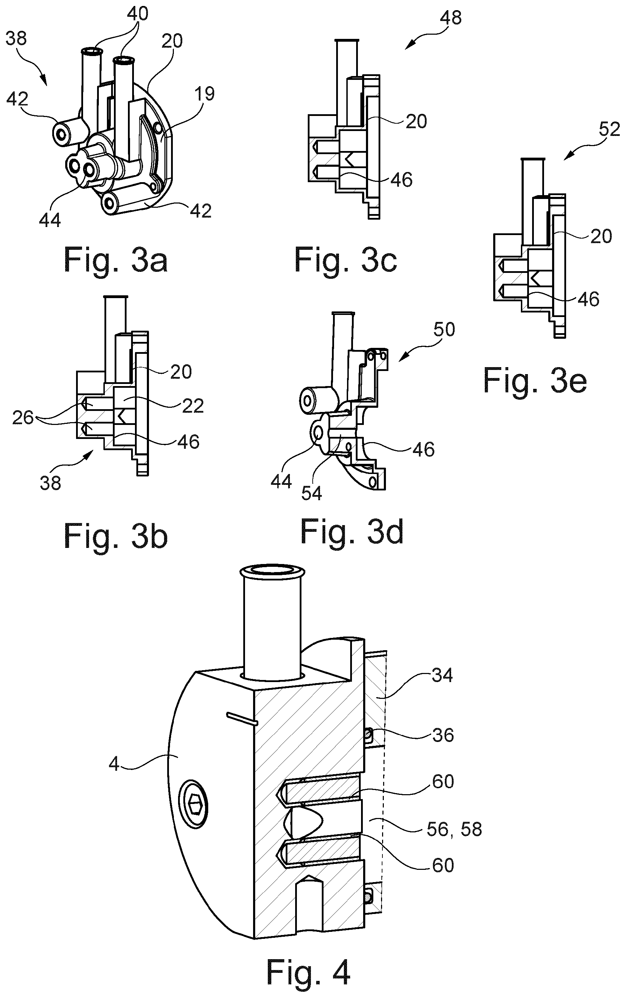

Fig. 3a zeigt einen Universal-Pumpengehäuserohling oder Universal-Pumpengehäuseabschnittrohling 38 vor seiner Weiterverarbeitung. Der Universal-Pumpengehäuserohling 38 weist die im Wesentlichen runde Grundplatte 19 mit dem Anschlussflansch 20 auf der einen Seite der Grundplatte 19 auf. Auf der gegenüberliegenden Seite des Anschlussflanschs 20 sind verschiedene Anschlüsse ausgebildet. Der Universal-Pumpengehäuserohling 38 weist zwei Schlauchtüllen 40 zum Anschließen von Schläuchen (nicht dargestellt) auf. Ferner weist der Universal-Pumpengehäuserohling 38 zwei Befestigungsstutzen 42 zum Anschrauben/Befestigen des Rückschlagventils 2 und zwei Anschlussstutzen 44 zum Anschließen des Rückschlagventils 2 auf. Bei dem Universal-Pumpengehäuserohling 38 vor der Weiterverarbeitung sind die Anschlussstutzen 44 nicht durch- oder aufgebohrt,bzw. nicht durchgängig, d.h. es besteht keine Fluidverbindung zwischen den Anschlussstutzen 44 und dem Inneren der Aufnahmetasche 22.

Fig. 3b zeigt eine Schnittansicht des Universal-Pumpengehäuserohlings 38 in Fig. 3a. Der Universal-Pumpengehäuserohling 38 weist die zwei Aufnahmebohrungen 26 auf, in denen die Zahnräder über die Zylinderstifte (in Fig. 3b nicht gezeigt) gelagert sind. Der Universal-Pumpengehäuserohling 38 weist ferner die Aufnahmetasche 22 für die zwei Zahnräder 16, 18 (in Fig. 3b nicht gezeigt) auf. Die Zahnräder 16, 18 sitzen auf einer Zahnrad-Anlagefläche 46, d.h. die Zahnräder 16, 18 gleiten beim Rotieren an der Zahnrad-Anlagefläche 46 entlang. Der Universal-Pumpengehäuserohling 38 ist dazu vorbereitet, durch spanende Bearbeitung in einen von drei Varianten des Pumpengehäuses oder Pumpengehäuseabschnitts 4 umgewandelt zu werden.

Die drei Varianten sind ein Pumpengehäuseabschnitt für eine Entgasungspumpe 48, ein Pumpengehäuseabschnitt für eine Dialysierflüssigkeit-Eingangspumpe 50 und ein Pumpengehäuseabschnitt für eine Dialysierflüssigkeit-Ausgangspumpe 52. Der Pumpengehäuseabschnitt für die Entgasungspumpe 48, der in Fig. 3c gezeigt ist, und der Pumpengehäuseabschnitt für die Dialysierflüssigkeit-Ausgangspumpe 52, der in Fig. 3e gezeigt ist, unterscheiden sich lediglich durch die Tiefe der Aufnahmetasche 22 für die Zahnräder 16, 18 voneinander. Eine Entgasungspumpe ist auf eine höhere Leistung als die anderen Pumpen 1 ausgelegt und benötigt deshalb Zahnräder 16, 18 mit einer höheren Zahnraddicke. Für die dickeren Zahnräder 16, 18 weist der Pumpengehäuseabschnitt für die Entgasungspumpe 48 eine tiefere Aufnahmetasche 22 auf als der Pumpengehäuseabschnitt für die Dialysierflüssigkeit-Ausgangspumpe 52. Der Pumpengehäuseabschnitt für die Dialysierflüssigkeit-Eingangspumpe 50 ist in Fig. 3d gezeigt und hat die gleiche Dimensionierung wie der Pumpengehäuseabschnitt für die Dialysierflüssigkeit-Ausgangspumpe 52. D.h. die Aufnahmetasche 22 des Pumpengehäuseabschnitts für die Dialysierflüssigkeit-Eingangspumpe 50 ist genauso tief wie die Aufnahmetasche 21 des Pumpengehäuseabschnitts für die Dialysierflüssigkeit-Ausgangspumpe 52. Ein Unterschied bei dem Pumpengehäuseabschnitt für die Dialysierflüssigkeit-Eingangspumpe 50 liegt darin, dass ein oder mehrere Durchgangskanäle 54 von der Zahnrad-Anlagefläche 46 zu den Anschlussstutzen 44 aufgebohrt ist oder sind. Durch die Durchgangskanäle 54 steht die Zahnrad-Anlagefläche 46 mit dem Rückschlagventil 2 der Dialysierflüssigkeit-Eingangspumpe in fluidischem Kontakt.

Fig. 4 zeigt einen Ausschnitt von einem Querschnitt durch eine Pumpe 1 nach einer weiteren Ausführungsform. Zwei Zahnräder 56, 58 sind auf Gleitlagern 60 in dem Pumpengehäuse oder Pumpengehäuseabschnitt 4 gelagert. Dazu müssen lediglich die Lagerpassungen und die Wandstärken angepasst werden. Dabei sind die Zahnräder 56, 58 nicht auf Zylinderstiften gelagert, wie in der ersten Ausführungsform. Vielmehr weisen die Zahnräder 56, 58 einen hervorstehenden Stutzen 61 auf. Es ist weiterhin vorstellbar, die Zahnräder 56, 58 auf gleitenden Vollwellen (nicht dargestellt) in dem Pumpengehäuseabschnitt 4 zu lagern. Bei beiden Varianten muss die Schmierung der Lager sichergestellt werden.

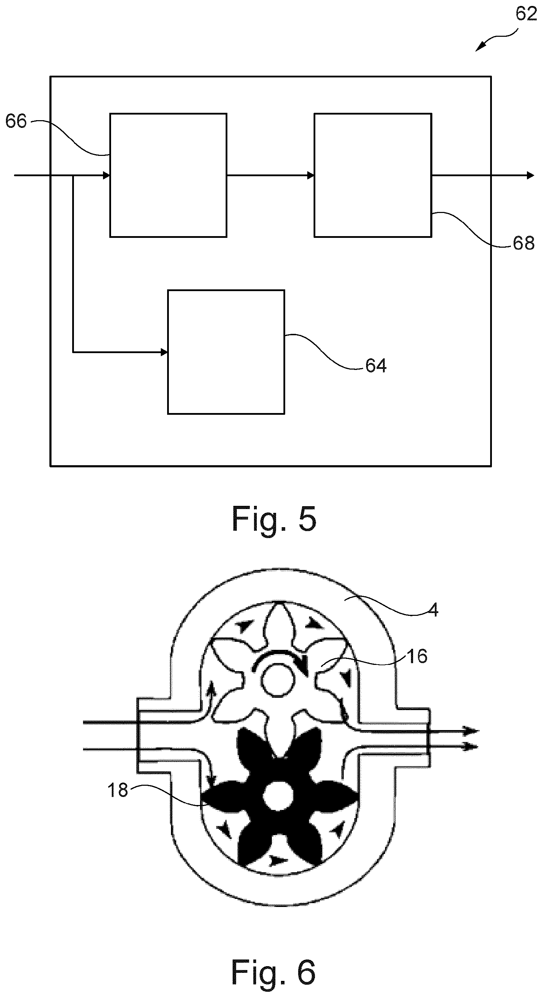

Fig. 5 zeigt eine schematische Ansicht einer offenbarungsgemäßen Dialysemaschine 62. Die Dialysemaschine 62 ist eine Blutbehandlungsvorrichtung zur extrakorporalen Behandlung von Blut. Die Dialysemaschine 62 weist einen Blutkreislauf und einen Dialysierflüssigkeits-Kreislauf auf. Der Dialysierflüssigkeits-Kreislauf weist drei unterschiedliche Pumpen 1 auf. Die erste Pumpe ist eine Entgasungspumpe 64, die zweite Pumpe ist eine Dialysierflüssigkeit-Eingangspumpe 66 und die dritte Pumpe ist eine Dialysierflüssigkeit-Ausgangspumpe 68. Die Entgasungspumpe 64 ist dafür vorgesehen die Dialysierflüssigkeit zu entgasen. Die Dialysierflüssigkeit-Eingangspumpe 66 und die Dialysierflüssigkeit-Ausgangspumpe 68 sind zur Bilanzierung der Dialysierflüssigkeit während der Blutbehandlung vorgesehen. Die unterschiedlichen Pumpen 1 haben jeweils unterschiedliche Anforderungen. Die Entgasungspumpe 64 muss eine höhere Leistung liefern als die anderen Pumpen. D.h. der Volumenstrom, den die Entgasungspumpe 64 liefern muss, ist größer als bei den anderen Pumpen. Daher weist die Entgasungspumpe 64 Zahnräder 16, 18 mit einer höheren Zahnraddicke auf. Die Dialysierflüssigkeit-Ausgangspumpe 68 ist baugleich zu der Entgasungspumpe 64 mit Ausnahme der Zahnraddicke. Die Zahnräder 16, 18 der Dialysierflüssigkeit-Ausgangspumpe 68 sind dünner als die der Entgasungspumpe 64. Die Dialysierflüssigkeit-Eingangspumpe 66 hat die gleiche Zahnraddicke wie die Dialysierflüssigkeit-Ausgangspumpe 68. Die Dialysierflüssigkeit-Eingangspumpe 66 weist aber das Rückschlagventil 2 auf, das bei Überdruck öffnet und die Dialysemaschine 62 vor zu hohen Drücken schützt. Bei der Dialysierflüssigkeit-Eingangspumpe 66 wird ein oder mehrere Durchgangskanäle 54 zwischen der Aufnahmetasche 22 der Dialysierflüssigkeit-Eingangspumpe 66 und dem Äußeren der Pumpe aufgebohrt. Auf diesen Durchgangskanal 54 wird das Rückschlagventil 2 aufgesetzt.

Die Entgasungspumpe 64 weist Zahnräder 16, 18 mit einer Breite von vorzugsweise 10,5 mm auf. Die Dialysierflüssigkeit-Ausgangspumpe 68 dagegen hat Zahnräder 16, 18 mit einer Breite von vorzugsweise 9,5 mm. An der Dialysierflüssigkeit-Eingangspumpe 66 wird das zusätzliche Rückschlagventil 2 angebracht, welches bei zu hohem Druck öffnet und die Dialysemaschine 62 vor Überlast/Überdruck schützt. Der Universal-Pumpengehäuserohling 38 ist dabei für alle Pumpen 1 identisch. Der Universal-Pumpengehäuserohling 38 muss lediglich leicht spanend für die jeweilige Anwendung bearbeitet werden. Für die Entgasungspumpe 64 wird die Aufnahmetasche 22 auf eine Tiefe von vorzugsweise 10,5 mm gefräst. Für die Dialysierflüssigkeit-Ausgangspumpe 68 und die Dialysierflüssigkeit-Eingangspumpe 66 wird die Aufnahmetasche 22 auf eine Tiefe von vorzugsweise 9,5mm gefräst. Für die Dialysierflüssigkeit-Eingangspumpe 66 werden zusätzliche Gewinde 70 an den Befestigungsstutzen 42 und Durchgangskanäle 54 zur Aufnahme des Rückschlagventils 2 eingebracht.

Für die Entgasungspumpe 64 werden in den Universal-Pumpengehäuserohling 38 die Zahnrad-Anlagefläche 46, Lagerpassungen und Dichtflächen eingefräst. Für die Montage des Spalttopfes 32 werden Gewinde 30 eingebracht. Die Aufnahmetasche 22 wird auf 10,5 mm aufgebohrt. Für die Dialysierflüssigkeit-Ausgangspumpe 68 und Dialysierflüssigkeit-Eingangspumpe 66 erfolgen dieselben Bearbeitungsschritte. Lediglich die Tiefe der Aufnahmetasche wird auf 9,5 mm bearbeitet. In die Dialysierflüssigkeit-Eingangspumpe 66 wird zur Aufnahme des Rückschlagventils 2 in den Außenaufnahmen Gewinde 70 eingebracht und die Durchgangskanäle 54 von den Zahnrädern 16, 18 zum Rückschlagventil 2 aufgebohrt.

Fig. 6 zeigt die zwei miteinander kämmenden Zahnräder 16, 18. Die zwei Zahnräder 16, 18 sind in dem Pumpengehäuse oder Pumpengehäuseabschnitt 4 angeordnet. Durch die Rotation der Zahnräder 16, 18 wird das Fluid, vorzugsweise die Dialysierflüssigkeit, gefördert.

Die Aufgabe der Offenbarung kann nicht nur durch eine Zahnradpumpe mit den Zahnrädern 16, 18 als Fluidförderungseinrichtung gelöst werden. Es ist weiterhin möglich eine Zahnringpumpe oder eine Innenzahnradpumpe zu verwenden. Auch bei einer Innenzahnradpumpe hängt die Förderleistung der Pumpe beispielsweise von der Zahnraddicke ab. Dadurch kann durch einen Universal-Pumpengehäuserohling 38 mit variierbarer Tiefe der Aufnahmetasche für die Zahnräder auch bei der Innenzahnradpumpe die Leistung variiert werden.

### Bezugszeichenliste

- 1: Pumpe
- 2: Rückschlagventil
- 4: Pumpengehäuseabschnitt
- 6: elektrischer Antrieb
- 8: Rotor Welle
- 10: Antriebswelle
- 12: Magnetkupplung
- 14: Antriebsgehäuse
- 16: erstes Zahnrad
- 18: zweites Zahnrad
- 20: Anschlussflansch
- 22: Aufnahmetasche
- 24: Zylinderstift
- 26: Aufnahmebohrung
- 28: Montageflansch
- 30: Gewinde
- 34: Dichtplatte
- 38: Universal-Pumpengehäuserohling
- 40: Schlauchtüllen
- 44: Anschlussstutzen
- 46: Zahnrad-Anlagefläche
- 48: Entgasungspumpe
- 50: Dialysierflüssigkeit-Eingangspumpe
- 52: Dialysierflüssigkeit-Ausgangspumpe
- 54: Durchgangskanäle
- 56: Zahnrad
- 58: Zahnrad
- 60: Gleitlager
- 62: Dialysemaschine
- 64: Entgasungspumpe
- 66: Dialysierflüssigkeit-Eingangspumpe
- 68: Dialysierflüssigkeit-Ausgangspumpe

## Patentansprüche

1. Verfahren zum Herstellen von Pumpen (1) unterschiedlicher Leistung zur Verwendung in einer Blutbehandlungsvorrichtung (62), vorzugsweise Dialysemaschine, mit einem Pumpengehäuse oder Pumpengehäuseabschnitt (4) zur lagernden Aufnahme einer Fluidfördereinrichtung (16, 18), vorzugsweise zweier in Kämmeingriff befindlicher Zahnräder, mit den folgenden Schritten:
- Urformen eines Universal-Pumpengehäuserohlings oder Universal-Pumpengehäuseabschnittrohlings (38) mit allen, den unterschiedlichen Pumpen (1) gemeinsamen Gehäusemerkmalen sowie mit solchen Übermaßen, die eine spanende Bearbeitung zur Herstellung aller unterschiedlichen Pumpen (1) aus demselben urgeformten Universal-Pumpengehäuserohling oder Universal-Pumpengehäuseabschnittrohling (38) erlauben und
- Individualisieren des Universal-Pumpengehäuserohlings oder Universal-Pumpengehäuseabschnittrohlings (38) durch spanabhebende Bearbeitung im Bereich der Übermaße zur Erreichung der jeweiligen Leistung.

2. Verfahren nach Anspruch 1, **dadurch gekennzeichnet, dass** beim Individualisieren des Universal-Pumpengehäuserohlings oder Universal-Pumpengehäuseabschnittrohlings (38) Aufnahmetaschen (22) für die Fluidfördereinrichtung (16, 18) derart ausgebildet werden, insbesondere durch spanende Bearbeitung vergrößert oder verkleinert werden, dass jene, entsprechende Abmessungen aufweisende Fluidfördereinrichtung (16, 18) aufnehmbar ist, wobei die Fluidfördereinrichtung (16, 18) die jeweilig geforderte Leistung bereitstellt.

3. Verfahren nach Anspruch 1 oder 2, **dadurch gekennzeichnet, dass** beim Individualisieren des Universal-Pumpengehäuserohlings oder Universal-Pumpengehäuseabschnittrohlings (38) Zahnrad-Auflageflächen (46) der Aufnahmetaschen (22) spanend radial abgetragen werden, um einen Durchmesser der jeweiligen Aufnahmetaschen (22) zu vergrößern.

4. Verfahren nach einem der Ansprüche 1 bis 3, **dadurch gekennzeichnet, dass** beim Individualisieren des Universal-Pumpengehäuserohlings oder Universal-Pumpengehäuseabschnittrohlings (38) die axialen Innenseiten der Aufnahmetaschen (22) axial spanend abgetragen werden, um eine Tiefe der Aufnahmetaschen (22) zu vergrößern.

5. Verfahren nach Anspruch 1 oder 2, **dadurch gekennzeichnet, dass** eine axiale Erstreckung des Universal-Pumpengehäuserohlings oder Universal-Pumpengehäuseabschnittrohlings (38) ein Übermaß aufweist und zur Verkleinerung, insbesondere axiale Verkürzung, der Aufnahmetaschen (22) axial spanend gekürzt wird.

6. Verfahren nach Anspruch 5, **gekennzeichnet durch** einen am Universal-Pumpengehäuserohling oder Universal-Pumpengehäuseabschnittrohling (38) axial ausgerichteten Anschlussflansch (20), an den ein Antrieb über einen dort ausgebildeten Montageflansch (28) angeschlossen wird, wobei am Anschlussflansch (20) des Universal-Pumpengehäuserohlings oder Universal-Pumpengehäuseabschnittrohlings (38) das Übermaß ausgebildet ist, welches axial abgefräst wird, um die Aufnahmetaschen (22) zu verkleinern.

7. Verfahren nach Anspruch 6, **dadurch gekennzeichnet, dass** der Anschlussflansch (20) und der Montageflansch (28) unter Zwischenfügen ausschließlich von Dichtungen (32, 34, 36) sowie ohne zusätzliche Distanzringe miteinander verbunden werden.

8. Verfahren nach einem der Ansprüche 1 bis 7, **dadurch gekennzeichnet, dass** beim Urformen des Universal-Pumpengehäuserohlings oder Universal-Pumpengehäuseabschnittrohlings (38) Schlauchtüllen (40) geformt werden, die integral mit dem Universal-Pumpengehäuserohling oder Universal-Pumpengehäuseabschnittrohling (38) ausgebildet sind.

9. Pumpe (1) zur Verwendung in einer Blutbehandlungsvorrichtung (62) vorzugsweise Dialysemaschine, aufweisend:
- einen elektrischen Antrieb (6), insbesondere Elektromotor, mit einem auf einer Antriebswelle (10) montierten oder ausgebildeten Rotor (8), der in einem Antriebsgehäuse (14) gelagert ist, welches einen Montageflansch (28) hat,
- ein Pumpengehäuse oder Pumpengehäuseabschnitt (4) mit einem Anschlussflansch (20), der nach dem Verfahren gemäß einem der Ansprüche 1 bis 8 durch Individualisierung des Universal-Pumpengehäuserohlings oder Universal-Pumpengehäuseabschnittrohlings (38) hergestellt und an den Montageflansch (28) angeflanscht ist, derart, dass die Antriebswelle (10) in einen von den Aufnahmetaschen (22) gemeinsam ausgebildeten Innenraum des Pumpengehäuses oder Pumpengehäuseabschnitts (4) ragt, wobei der Anschlussflansch (20) und der Montageflansch (28) ausschließlich unter Zwischenfügen von Dichtungen (32, 34, 36) ohne zusätzliche Distanzringe zur individuellen Einstellung der axialen Abmessung der Aufnahmetaschen (22) miteinander verbunden sind, und
- die Fluidfördereinrichtung (16, 18) in Form zweier in Kämmeingriff befindlicher Zahnräder, die in Wirkeingriff mit der Antriebswelle (10) stehen und an den Innenseiten der Aufnahmetaschen (22) abgleiten.

10. Pumpe (1) nach Anspruch 9 oder 10, **dadurch gekennzeichnet, dass** die Zahnräder (16, 18; 56, 58) auf Zylinderstiften (24) gelagert sind, die in dem Pumpengehäuse oder Pumpengehäuseabschnitt (4) verpresst sind.

11. Pumpe (1) nach einem der Ansprüche 9 bis 11, **dadurch gekennzeichnet, dass** die Zahnräder (16, 18; 56, 58) in dem Pumpengehäuse oder Pumpengehäuseabschnitt mit Gleitlagern (60) gelagert sind.

12. Pumpe (1) nach einem der Ansprüche 9 bis 12, **dadurch gekennzeichnet, dass** die Zahnräder (16, 18; 56, 58) auf einer direkt gleitenden Vollwelle in dem Pumpengehäuse oder Pumpengehäuseabschnitt (4) gelagert sind.

13. Dialysemaschine (62) zum extrakorporalen Reinigen von Blut mit mehreren Pumpen (1) unterschiedlicher Leistung, die sämtlich nach dem Verfahren gemäß einem der Ansprüche 1 bis 7 hergestellt sind.

## Claims

1. A method of manufacturing pumps (1) of different performances for use in a blood treatment device (62), preferably a dialysis machine, having a pump housing or a pump housing portion (4) for supportingly holding a fluid delivery device (16, 18), preferably two gear wheels in meshing engagement, comprising the following steps:
- primary shaping of a universal pump housing blank or a universal pump housing portion blank (38) with all the housing features common to the different pumps (1) as well as with such oversizes as to allow machining for manufacturing all different pumps (1) from the same primary-shaped universal pump housing blank or universal pump housing portion blank (38), and
- individualizing the universal pump housing blank or universal pump housing portion blank (38) by machining in the region of the oversizes to achieve the respective performance.

2. The method according to claim 1, **characterized in that,** during individualizing the universal pump housing blank or universal pump housing portion blanks (38), receiving pockets (22) for the fluid delivery device (16, 18) are configured, in particular enlarged or reduced by machining, in such a way that the fluid delivery device (16, 18) having corresponding dimensions can be accommodated, wherein the fluid delivery device (16, 18) provides the respectively required performance.

3. The method according to claim 1 or 2, **characterized in that** during individualizing the universal pump housing blank or universal pump housing portion blank (38), gear wheel support surfaces (46) of the receiving pockets (22) are machined radially in order to increase a diameter of the respective receiving pockets (22).

4. The method according to one of claims 1 to 3 **characterized in that** during individualization of the universal pump housing blank or universal pump housing portion blank (38), the axial inner sides of the receiving pockets (22) are axially machined off in order to increase a depth of the receiving pockets (22).

5. The method according to claim 1 or 2, **characterized in that** an axial extension of the universal pump housing blank or universal pump housing portion blank (38) has an oversize and is shortened by axial machining to reduce the size of the receiving pockets (22), in particular to axially shorten it.

6. The method of claim 5, **characterized by** a connecting flange (20) axially oriented with the universal pump housing blank or universal pump housing portion blank (38) and to which a drive is connected via a mounting flange (28) configured there, wherein the oversize is configured on the connecting flange (20) of the universal pump housing blank or universal pump housing portion blank (38) and which is axially milled off to reduce the receiving pockets (22).

7. The method according to claim 6, **characterized in that** the connecting flange (20) and the mounting flange (28) are connected to each other exclusively by interposing seals (32, 34, 36) as well as without additional spacer rings.

8. The method according to one of claims 1 to 7, **characterized in that** when the universal pump housing blank or universal pump housing portion blank (38) is primary molded, hose grommets (40) are formed that are integrally formed with the universal pump housing blank or universal pump housing portion blank (38).

9. A pump (1) for use in a blood treatment device (62), preferably a dialysis machine, comprising:
- an electric drive (6), in particular an electric motor with a rotor (8) mounted or formed on a drive shaft (10) and supported in a drive housing (14) having a mounting flange (28),
- a pump housing or pump housing portion (4) with a connecting flange (20), which is manufactured according to the method according to one of the claims 1 to 8 by individualizing the universal pump housing blank or universal pump housing portion blank (38) and is flanged to the mounting flange (28), in such a way that the drive shaft (10) projects into an interior space of the pump housing or pump housing portion (4) formed jointly by the receiving pockets (22), wherein the connecting flange (20) and the mounting flange (28) are connected to each other exclusively by interposing seals (32, 34, 36) without additional spacer rings for individual adjustment of the axial dimension of the receiving pockets (22), and
- the fluid delivery device (16, 18) in the form of two gear wheels in meshing engagement, which are in operative engagement with the drive shaft (10) and slide against the inner sides of the receiving pockets (22).

10. The pump (1) according to claim 9 or 10, **characterized in that** the gear wheels (16, 18; 56, 58) are mounted on cylindrical pins (24) that are pressed in the pump housing or pump housing portion (4).

11. The pump (1) according to one of claims 9 to 11, **characterized in that** the gear wheels (16, 18; 56, 58) are mounted in the pump housing or pump housing portion with slide bearings (60).

12. The pump (1) according to one of claims 9 to 12, **characterized in that** the gear wheels (16, 18; 56, 58) are mounted on a directly sliding solid shaft in the pump housing or pump housing portion (4).

13. A dialysis machine (62) for extracorporeal purification of blood comprising a plurality of pumps (1) of different performance, all of which are manufactured by the method according to one of claims 1 to 7.

## Revendications

1. Procédé de fabrication de pompes (1) de différente puissance pour l'utilisation dans un dispositif de traitement du sang (62), de préférence une machine de dialyse, avec un boîtier de pompe ou une section de boîtier de pompe (4) pour le logement de stockage d'un dispositif de transport de fluide (16, 18), de préférence de deux roues dentées se trouvant en prise d'engrènement, avec les étapes suivantes :
- le formage initial d'une ébauche de boîtier de pompe universelle ou d'une ébauche de section de boîtier de pompe universelle (38) avec toutes les caractéristiques de boîtier communes aux différentes pompes (1) ainsi qu'avec de tels surdimensionnements qui permettent un usinage par enlèvement de copeaux pour fabriquer toutes les pompes différentes (1) à partir de la même ébauche de boîtier de pompe universelle ou ébauche de section de boîtier de pompe universelle (38) formée initialement et
- l'individualisation de l'ébauche de boîtier de pompe universelle ou de l'ébauche de section de boîtier de pompe universelle (38) par usinage par enlèvement de copeaux dans la zone des surdimensionnements pour atteindre la puissance respective.

2. Procédé selon la revendication 1, **caractérisé en ce que**, lors de l'individualisation de l'ébauche de boîtier de pompe universelle ou de l'ébauche de section de boîtier de pompe universelle (38), des poches de réception (22) pour le dispositif de transport de fluide (16, 18) sont formées, en particulier sont agrandies ou réduites par usinage par enlèvement de copeaux de sorte que ce dispositif de transport de fluide (16, 18) présentant des dimensions correspondantes puisse être reçu, dans lequel le dispositif de transport de fluide (16, 18) fournit la puissance respectivement requise.

3. Procédé selon la revendication 1 ou 2, **caractérisé en ce que**, lors de l'individualisation de l'ébauche de boîtier de pompe universelle ou de l'ébauche de section de boîtier de pompe universelle (38), des surfaces d'appui de roue dentée (46) des poches de réception (22) sont enlevées radialement par enlèvement de copeaux afin d'augmenter un diamètre des poches de réception (22) respectives.

4. Procédé selon l'une quelconque des revendications 1 à 3, **caractérisé en ce que**, lors de l'individualisation de l'ébauche de boîtier de pompe universelle ou de l'ébauche de section de boîtier de pompe universelle (38), les côtés intérieurs axiaux des poches de réception (22) sont enlevés axialement par enlèvement de copeaux afin d'augmenter une profondeur des poches de réception (22).

5. Procédé selon la revendication 1 ou 2, **caractérisé en ce qu'**une étendue axiale de l'ébauche de boîtier de pompe universelle ou de l'ébauche de section de boîtier de pompe universelle (38) présente un surdimensionnement et est raccourcie par enlèvement de copeaux axialement pour réduire, en particulier raccourcir axialement, les poches de réception (22).

6. Procédé selon la revendication 5, **caractérisé par** une bride de raccordement (20) orientée axialement au niveau de l'ébauche de boîtier de pompe universelle ou de l'ébauche de section de boîtier de pompe universelle (38), à laquelle un entraînement est raccordé par le biais d'une bride de montage (28) qui y est formée, dans lequel le surdimensionnement est formé au niveau de la bride de raccordement (20) de l'ébauche de boîtier de pompe universelle ou de l'ébauche de section de boîtier de pompe universelle (38), surdimensionnement qui est fraisé axialement afin de réduire les poches de réception (22).

7. Procédé selon la revendication 6, **caractérisé en ce que** la bride de raccordement (20) et la bride de montage (28) sont reliées l'une à l'autre en intercalant exclusivement des joints d'étanchéité (32, 34, 36) et sans bagues d'écartement supplémentaires.

8. Procédé selon l'une quelconque des revendications 1 à 7, **caractérisé en ce que**, lors du formage initial de l'ébauche de boîtier de pompe universelle ou de l'ébauche de section de boîtier de pompe universelle (38), des embouts de tuyau (40) sont formés, lesquels sont formés d'un seul tenant avec l'ébauche de boîtier de pompe universelle ou l'ébauche de section de boîtier de pompe universelle (38).

9. Pompe (1) destinée à être utilisée dans un dispositif de traitement du sang (62) de préférence une machine de dialyse, présentant :
- un entraînement électrique (6), en particulier un moteur électrique, avec un rotor (8) monté ou formé sur un arbre d'entraînement (10), rotor qui est logé dans un boîtier d'entraînement (14) qui présente une bride de montage (28),
- un boîtier de pompe ou une section de boîtier de pompe (4) avec une bride de raccordement (20) qui est fabriquée selon le procédé selon l'une quelconque des revendications 1 à 8 par l'individualisation de l'ébauche de boîtier de pompe universelle ou l'ébauche de section de boîtier de pompe universelle (38) et est bridée au niveau de la bride de montage (28) de sorte que l'arbre d'entraînement (10) fasse saillie dans un espace intérieur formé avec les poches de réception (22) du boîtier de pompe ou de la section de boîtier de pompe (4), dans lequel la bride de raccordement (20) et la bride de montage (28) sont reliées l'une à l'autre exclusivement en intercalant des joints d'étanchéité (32, 34, 36) sans bagues d'écartement supplémentaires pour régler individuellement la dimension axiale des poches de réception (22) et
- le dispositif de transport de fluide (16, 18) sous la forme de deux roues dentées se trouvant en prise d'engrènement, roues qui sont en prise active avec l'arbre d'entraînement (10) et glissent sur les côtés intérieurs des poches de réception (22).

10. Pompe (1) selon la revendication 9 ou 10, **caractérisée en ce que** les roues dentées (16, 18 ; 56, 58) sont montées sur des tiges cylindriques (24) qui sont pressées dans le boîtier de pompe ou la section de boîtier de pompe (4).

11. Pompe (1) selon l'une quelconque des revendications 9 à 11, **caractérisée en ce que** les roues dentées (16, 18 ; 56, 58) sont montées avec des paliers lisses (60) dans le boîtier de pompe ou la section de boîtier de pompe.

12. Pompe (1) selon l'une quelconque des revendications 9 à 12, **caractérisée en ce que** les roues dentées (16, 18 ; 56, 58) sont montées sur un arbre plein glissant directement dans le boîtier de pompe ou la section de boîtier de pompe (4).

13. Machine de dialyse (62) pour le nettoyage extracorporel du sang avec plusieurs pompes (1) de différentes puissances qui sont toutes fabriquées selon le procédé selon l'une quelconque des revendications 1 à 7.
